# EUROPEAN PATENT SPECIFICATION

(11) **EP 2 790 763 B1**
(45) Date of publication and mention of the grant of the patent: **09.12.2015**
(21) Application number: 12809217.8
(22) Date of filing: 14.12.2012
(51) Int. Cl.: A61M 16/04

(54) **A LUNG VENTILATION DEVICE**
LUNGENBEATMUNGSVORRICHTUNG
DISPOSITIF DE VENTILATION PULMONAIRE

(30) Priority: 14.12.2011 IE 20110550
(43) Date of publication of application: 22.10.2014
(73) Proprietor: Cork Institute Of Technology, County Cork (IE)
(72) Inventor: DALY, Charles, Kanturk County Cork (IE); BRENNAN, Ciaran, Goresbridge Kilkenny (IE); O'BRIEN, Declan, Monkstown Cork (IE); MURRAY, Myles, Togher Cork (IE); O'DRISCOLL, Olive, Kinsale County Cork (IE)
(74) Representative: Weldon, Michael James
(86) International application number: PCT/EP2012/075556
(87) International publication number: WO 2013/087845

(56) References cited:
- GB-A- 2 373 445
- GB-A- 2 397 229
- US-A1- 2010 147 311

## Description

### INTRODUCTION

### Field of the Invention

The invention relates to ventilation of the lungs.

Ventilation of the lung is the process whereby gas enters and leaves the lung to allow exchange of oxygen and carbon dioxide between the alveoli and the blood. Normally this process is mechanically controlled by the action of the diaphragm. When the diaphragm contracts it descends and decreases intrathoracic pressure in the chest cavity, causing air to be pulled into the lungs through the trachea and bronchi.

When a patient is under general anaesthesia disruption can occur to normal breathing. A mechanical ventilator is used to ensure continuous breathing by controlled flow of gas into a patient's airways. The ventilator may be in complete control of the patient's breathing or may only be assisting, depending on the desired settings of the anaesthetist.

Gas is delivered from the ventilator using a tube which is placed directly into the patient's trachea/bronchus, depending on the surgical requirement. The plastics tube incorporates lumens to deliver the gases, and cuffs to seal onto the trachea or bronchus. This cuff prevents the leakage of anaesthetic gas into the atmosphere and operating theatre.

Most ventilation procedures in surgery are for the purposes of operating on a part of the body other than the lung or heart. A difficulty arises when it is necessary to operate on the lung itself, thereby posing the problem of operating on the very organ which is being used for ventilation. The answer is to have single lung ventilation to facilitate the surgery. Single lung ventilation is not only used for surgery on the lungs. More often now, several other surgical procedures can be performed by deliberately deflating one of the lungs to allow access to another organ in the mediastinum or chest cavity such as the heart or esophagus.

### Prior Art Discussion

US6513527 (Abdel-Aziz) and WO02/47748 (Bodenham), and US4646733 (Freudenberg) CN201469852U and JP2010017297 and NL1033898 describe a double lumen breathing tube for ventilating at least one lung of a patient.

GB2373445 (Bodenham) describes a bronchial ventilation device with three balloons.

GB2397229 (Ghosh) describes a bronchial device with a forked channel and a sealing balloon on the main stem.

While these devices provide for ventilation of both lungs during surgery, there is still a need for an improved device which can more reliably provide a ventilation passage for both lungs. More particularly, it is desirable to allow accurate device placement to be achieved more easily in the operating theatre, especially in emergency situations. Another objective is to achieve more device versatility.

### SUMMARY OF THE INVENTION

According to the invention, there is provided a lung ventilation device for human or animal treatment, the device comprising:
a stem configured to fit in a trachea,
said stem having at least two ventilation passageways communicating with distal passageways configured to fit in the bronchi at the carina, and
a positioning balloon configured to engage the walls at the carina when inflated, wherein:
   the positioning balloon has a proximal annular portion surrounding a distal end of the stem, and a plurality of annular distal portions, each surrounding a distal passageway end, and
   the distal balloon portions are extensions of the proximal balloon portion.

In one embodiment, the positioning balloon is configured to, when inflated, seal against the bronchi and the trachea.

In one embodiment, each distal balloon portion includes a turned-in wall which is integrated with a wall of the associated distal passageway.

In one embodiment, each ventilation passageway comprises a tube, and the balloon distal passageways are continuations of said ventilation passageway tubes.

In another embodiment, there is a frangible link between the distal passageways, said link being adapted to break to separate the distal passageways *in situ* for bronchi entry. Preferably, said link is adapted to break as the positioning balloon is inflated. In one embodiment, said link is also adapted to break upon contact with the carina.

In one embodiment, the frangible link is at or adjacent to the apex of an inverted V-shape formed by ends of said distal passageways.

In a further embodiment, the device further comprises a stylet extending along the stem and arranged to provide stiffness to the distal passageways during device advancement in the trachea. In one embodiment, there is a stylet for each ventilation passageway, the distal passageways have a default splayed-out configuration, and the stylets are arranged to retain the distal passageway together during tracheal advancement and to be retracted for separation at or near the carina.

In one embodiment, end faces of the distal passageways are slanted in a proximal and inward direction so that they form an inverted V-shape as the distal end of the ventilation device approaches the carina. In one embodiment, said configuration exists only when the distal passageways are retained together.

In one embodiment, the distal passageways have end faces which face outwardly at an acute angle to a longitudinal axis of the stem.

In one embodiment, the balloon is configured so that the action of inflation positions each distal passageway toward the centre of the bronchus.

In one embodiment, one distal balloon portion extends further distally than another distal balloon portion.

In one embodiment, the stem includes a lumen for liquid to be used during treatment. Preferably, said lumen is for radiopaque material.

In one embodiment, the proximal end and the stem of the device has a separate passageway corresponding to each distal passageway.

In one embodiment, the distal passageways are of a material which is more flexible than the stem.

In one embodiment, the device further comprises a proximal balloon around the stem and being proximal of the positioning balloon and a separate inflation means allowing inflation of the proximal balloon for an initial stage of dual lung inflation before inflation of the positioning balloon for one lung ventilation.

### DETAILED DESCRIPTION OF THE INVENTION

### Brief Description of the Drawings

The invention will be more clearly understood from the following description of some embodiments thereof, given by way of example only with reference to the accompanying drawings in which:-
Fig. 1 is a set of views of a ventilation device of the invention, including a front view, a side view, a cross-sectional view along the lines A-A, an enlarged view B of the distal end of the device, and a cross-sectional view C through the distal end, and Fig. 2 is a set of corresponding views showing the device with the balloon inflated;
Fig. 3 is a set of similar views of a ventilation device of another embodiment, in which a distal tip has a link element between the distal portions, Fig. 4 is a set of similar views in use showing this device being deployed by a surgeon as it is pushed towards the carina, Fig. 5 shows the device on the carina when the link element has opened and Fig. 6 shows the device when fully deployed with the cuff inflated at the carina;
Fig. 7 is a set of views similar to Fig. 1 of a ventilation device of an alternative embodiment, in this case having an additional, proximal inflatable cuff, and Fig. 8 is a set of views showing this device with the cuffs inflated;
Fig. 9 is a set of views of another device, the balloon being omitted for clarity, having four lumens and a stylet, the views being a front view, a longitudinal sectional view L-L, a perspective view of the stylet, a cross-sectional view J-J through the stem, and an enlarged view Detail A of the distal end of the device;
Fig. 10 is a front view and a cross-sectional view of an alternative device distal end, and again the balloon is omitted; and
Fig. 11 is a set of views of a device having a non-symmetrical distal end, the views being a front view, a side view, an enlarged view Detail B of the distal end showing internal details, and a two-dimensional external profile of the distal end.

### Description of the Embodiments

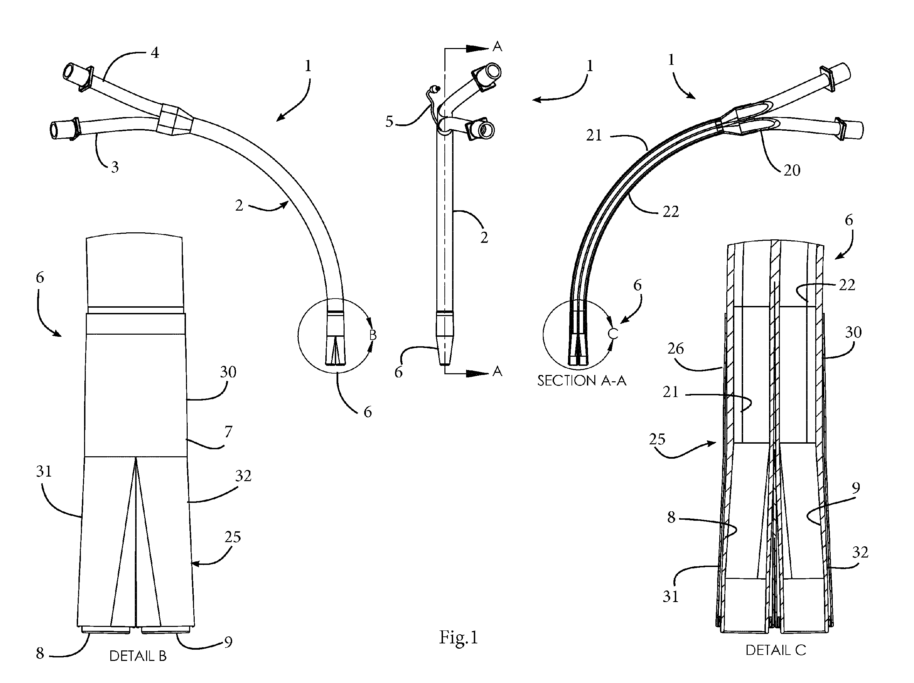

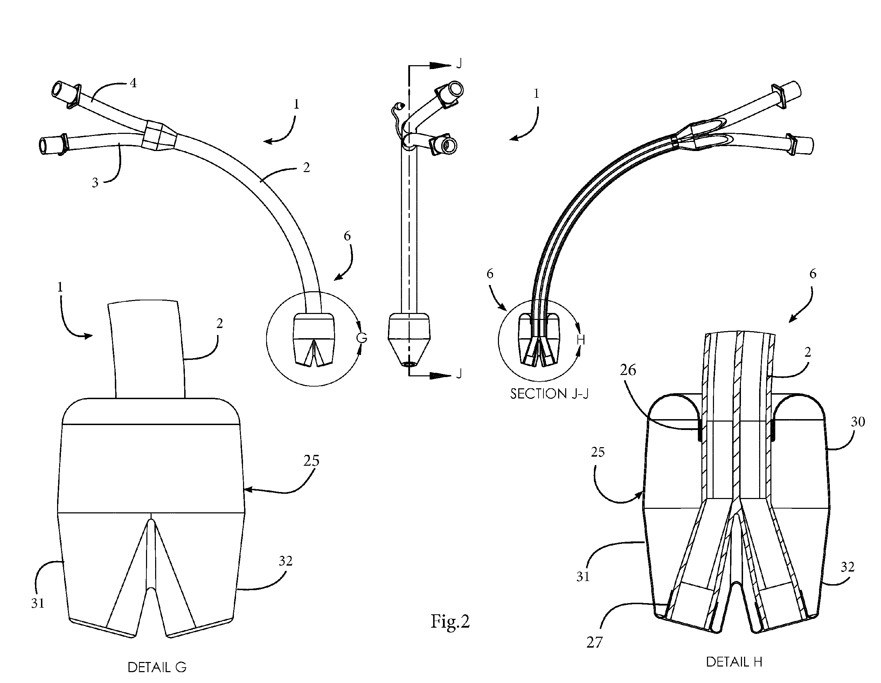

Referring to Figs. 1 and 2, a ventilation device 1 comprises a central stem 2 from which extends a pair of proximal ventilation tubes 3 and 4 for independent ventilation of the two lungs. At the proximal end there is also an inflation actuator 5 linked with a lumen which runs down through the device.

A distal portion 6 comprises a junction region 7 from which extend bronchi tubes 8 and 9 arranged to fit into the bronchi. The tubes 8 and 9 are continuations of tubes 21 and 22 in the stem 2, which are in turn continuations of the proximal tubes 3 and 4 respectively.

The distal portion 6 comprises a positioning balloon 25 which is sealed at its proximal end to the stem 2 by a circumferential seal 26, and terminates around the two distal tubes 8 and 9. There are two different attachments to the stem 2. Referring to detail H of Fig. 2, a proximal attachment seal 26 is folded over on itself, while a distal attachment 27 does not have any balloon material overlap. These are best viewed in Fig. 2 Detail H.

This arrangement provides in the balloon 25 an annular balloon region 30 surrounding the distal end of the stem 2. This is proportioned to engage and seal against the walls of the trachea when inflated. The balloon also has a pair of distal annular regions 31 and 32 surrounding the distal tubes 8 and 9. These regions increase in diameter in the proximal direction, where they join the proximal balloon region 30. Importantly, the balloon acts to seal the trachea and the bronchi, and it also provides structural strength without sacrificing flexibility. Hence, the device's distal portion 6 fits the carina without need for individual manipulation of the bronchi tubes 8 and 9. Additionally, when deflated the distal end 6 is compact for insertion but the action of inflation spreads the distal tubes 8 and 9 toward the centre of each bronchus, allowing for a seal between the balloon 25 and the carina.

The device 1 enables delivery of ventilation gas to both lungs, dual lung ventilation, or to the right or left lung for one lung ventilation of a person or animal. The device may be easily and quickly positioned correctly on the carina, even in emergency situations.

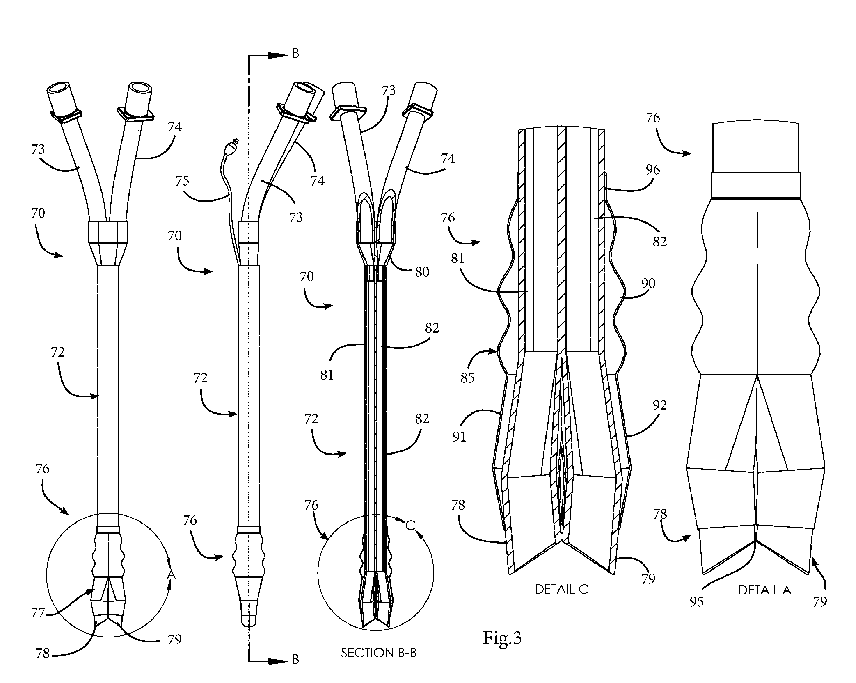

Referring to Fig. 3, a ventilation device 70 of another embodiment comprises a central stem 72 from which extend a pair of proximal ventilation tubes 73 and 74 for independent ventilation of the two lungs. At the proximal end there is also an inflation actuator 75 linked with an inflating lumen which runs through the stem 72.

A distal portion 76 comprises a junction region 77 and bronchi tubes 78 and 79 arranged to fit into the patient's bronchi B1 and B2. The tubes 78 and 79 are continuations of tubes 81 and 82 in the stem 72, which are in turn continuations of the proximal tubes 73 and 74 respectively.

The distal end 76 comprises a balloon 85 which is sealed at its proximal end to the stem 72 by a circumferential seal 96, and terminates and seals around the two distal tubes 78 and 79. This arrangement provides in the balloon 85 an annular proximal balloon region 90 surrounding the stem 72. This is proportioned to engage the walls of the trachea when inflated. The balloon 85 also has a pair of distal annular regions 91 and 92 surrounding the distal tubes 78 and 79. These balloon regions increase in diameter in the proximal direction, where they join the proximal region 90. There is a frangible seal 95 between the distal tubes 78 and 79.

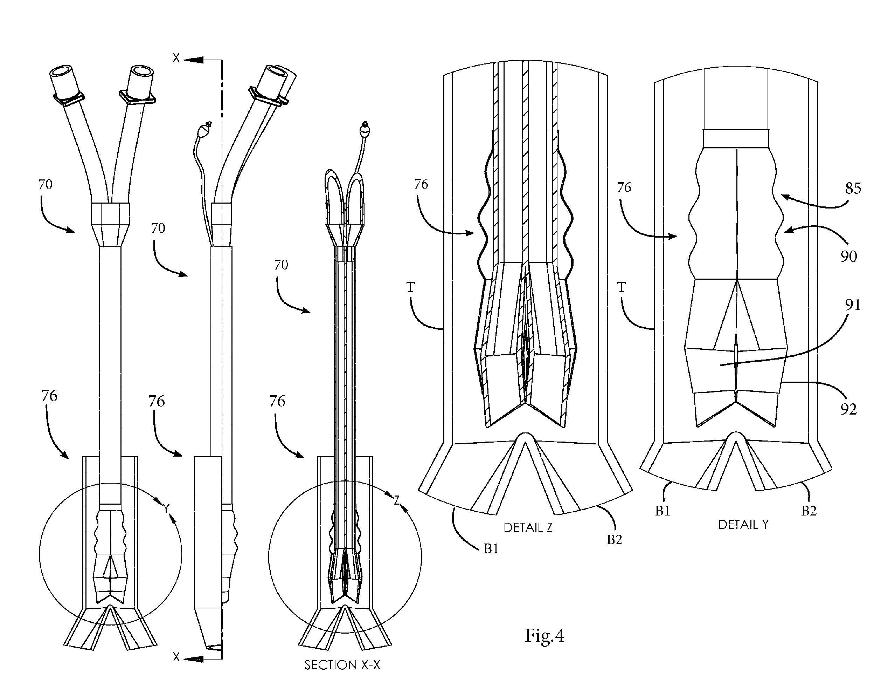

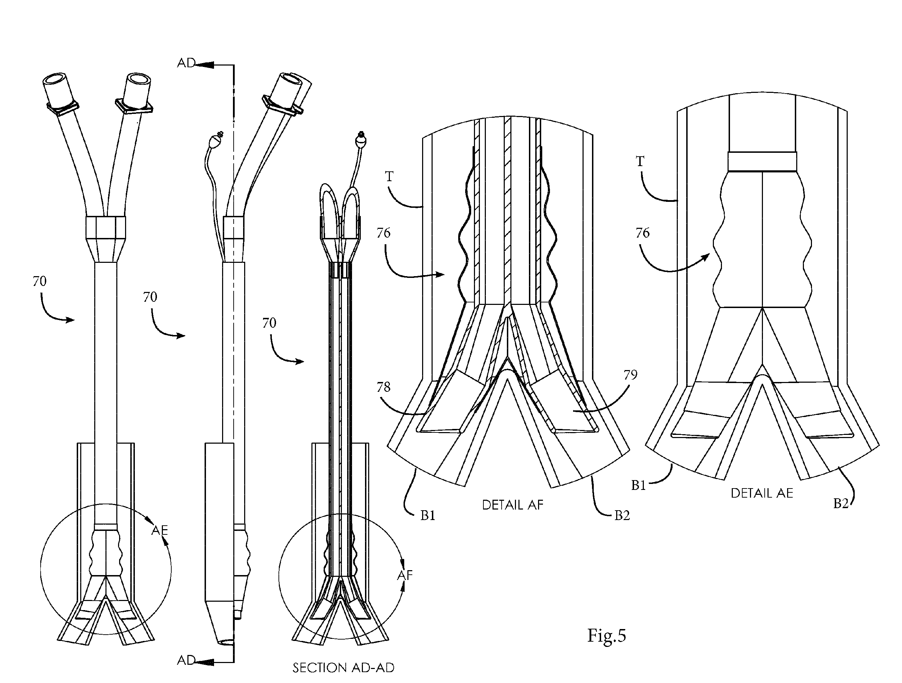

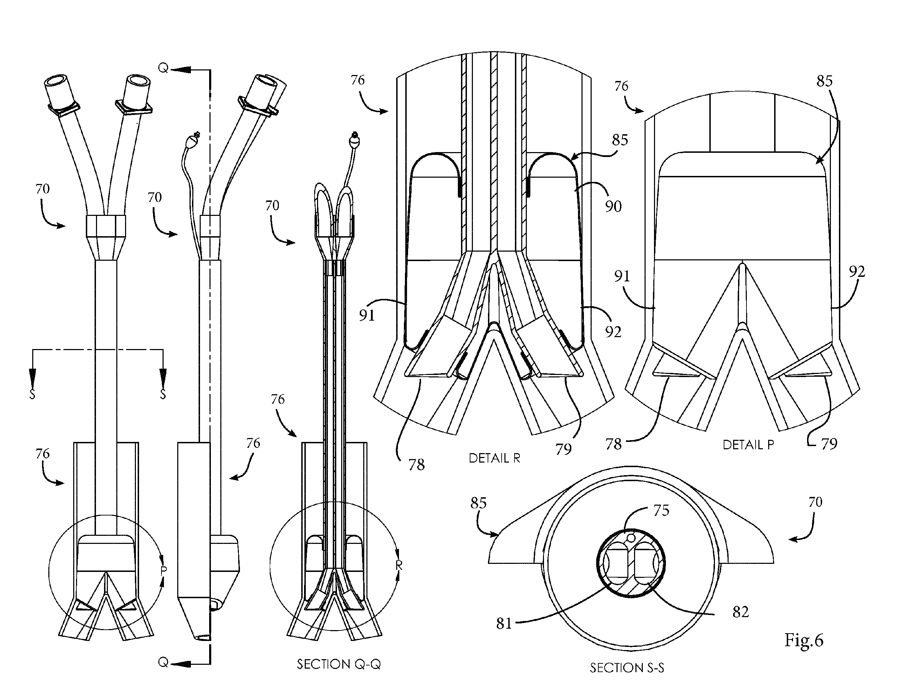

In use, as shown in Figs. 4, 5, and 6 the device 70 is pushed in the trachea T of a patient until the distal portion 76 reaches the carina junction with the bronchi B1 and B2. As the device 70 is pushed down the trachea T, when the carina is reached the seal 95 breaks due to balloon inflation, allowing the branches 78 and 79 to separate and enter the bronchi B1 and B2. Hence, the seal 95 performs the function of keeping the device 1 compact as it is moving down the trachea T. The end faces of the tubes 78 and 79 are sloped inwardly and proximally, thereby helping to centre the distal end 76 on the carina.

When the surgeon operates the actuator 75, causing the balloon 85 to inflate, the balloon region 90 engages the wall of the trachea T, and the regions 91 and 92 engage the walls of the bronchi B1 and B2. The inflation action helps to break the seal 95 and move the distal tubes 78 and 79 to the centre of the bronchi. Some further pushing down of the device 70 is required after breaking the seal 95. The balloon 85 thus anchors the device 71 to allow reliable ventilation via the tubes 73 and 74, with little risk of displacement upon movement of the patient. The configuration of the balloon 85 accepts external forces and dissipates them by means of an "accordion-like" configuration.

Importantly, the device is easily located at the carina, without need for the surgeon to manipulate individual tubes into the bronchi B1 and B2. Also, the configuration ensures that the device branches do not extend too far into the bronchi, possibly beyond take-off branches.

Also, the balloon arrangement seals each bronchus from the other bronchus and from the trachea.

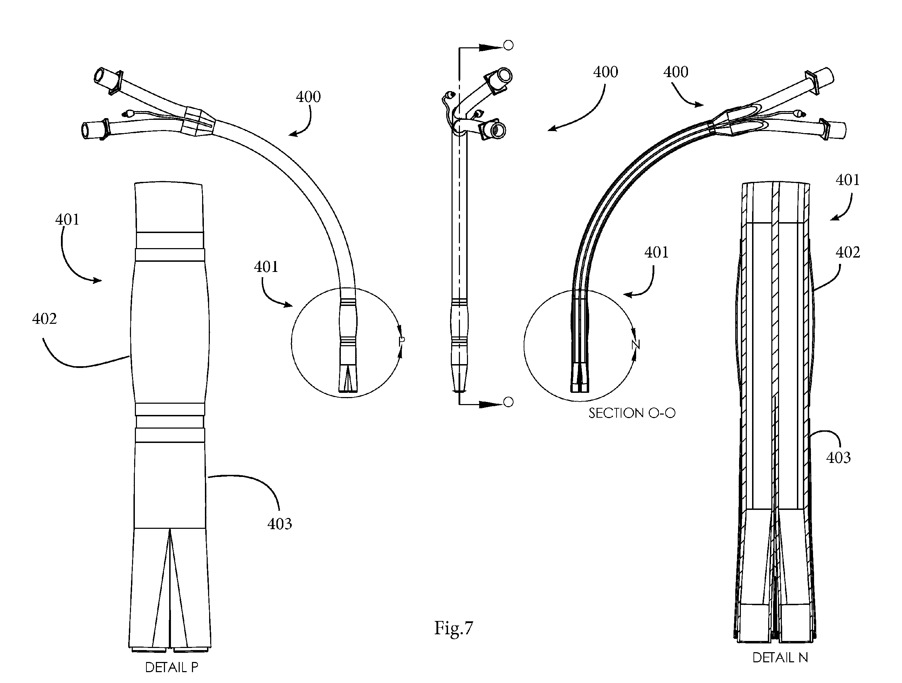

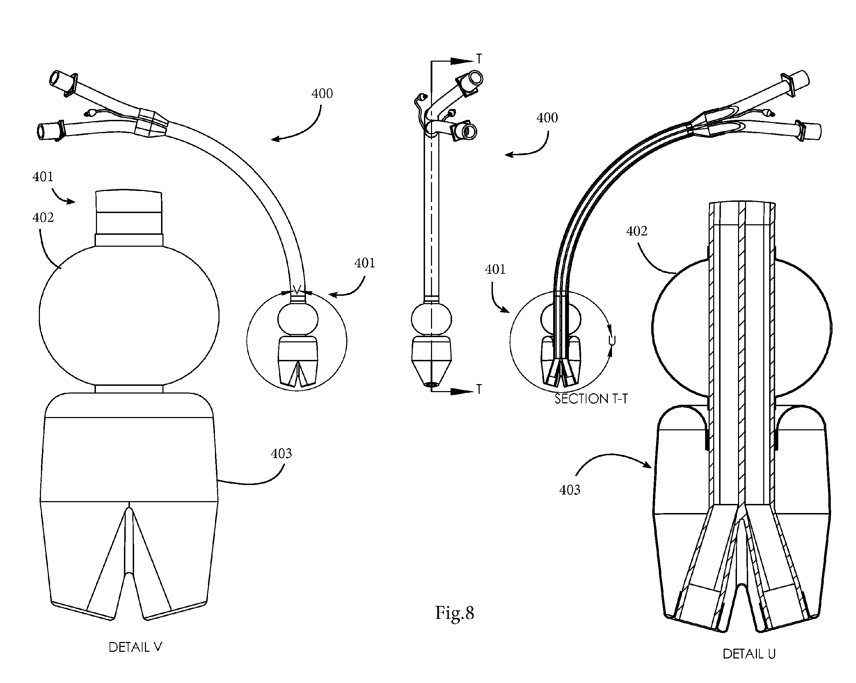

Figs. 7 and 8 show a device 400 having a distal portion 401 having proximal and distal balloons, namely a proximal balloon 402 and a positioning balloon 403. The purpose of the proximal balloon 402 is to allow placement and inflation in the trachea allowing dual lung ventilation. In this instance the distal balloon 403 is not inflated and the device has not advanced fully to the carina. This allows a faster intubation time, similar to standard endo-tracheal tubes. When lung isolation is required the proximal balloon 402 is deflated and the device 400 is advanced to the carina and the positioning balloon 403 is inflated. Thus, a single device can be used for dual lung ventilation and later one lung ventilation, reducing the need for tube change. It is expected that this hybrid aspect of the device 400 would save the tube exchange time, from endo tracheal to double lumen tube, and would be particularly beneficial for treating patients with difficult airways.

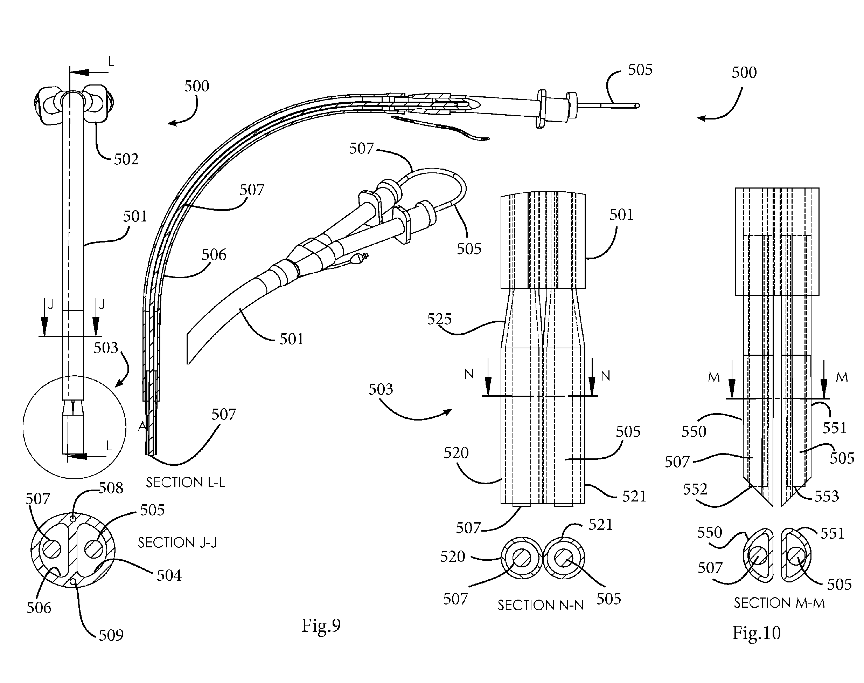

Referring to Fig. 9 a device 500 comprises a proximal end 502, a stem 501, and a distal end 503. As shown in the section J-J the stem 501 has:
ventilation tubes 504 and 506,
stylets 505 and 507 running through the ventilation tubes 504 and 506,
a balloon inflation lumen 508, and
a lumen 509 for radiopaque material.

For clarity, the balloon is not shown in these drawings; it is similar to the balloon 85 of the device 70.

Distally, the ventilation lumens 504 and 506 engage the distal portion 503 at a shoulder 525 leading to left and right bronchial legs 520 and 521. The distal portion 503 is of a material which is more flexible than the sleeve of the stem 501. This flexible material has a manufactured condition at a prescribed angle to the stem 501 to replicate the anatomical angle between the trachea and bronchus of the average patient. Because the distal portion 503 is of a more flexible material it allows movement to keep the profile low during insertion, allowing easier passage through the vocal chords.

Such flexibility can be achieved because stiffness during insertion is provided by the pair of stylets 505 and 507. When the stylet is partially withdrawn the legs return to the anatomical angle/manufactured condition allowing seating on the carina prior to inflation. Thus, when engaged, the stylets can advance to the distal end of the device, providing column strength while additionally holding the distal bronchial legs on the centreline allowing a low profile to pass through the vocal chords of the patient. The disengaged position moves the stylet 505/507 proximally relative to the ventilation tube allowing the distal bronchial legs to assume their original as-manufactured relaxed position for engagement with the patient carina and bronchi.

Referring to Fig. 10 an alternative distal end is illustrated. In this case there are also the stylets 505 and 507, and similar parts are given the same reference numerals. However, the stylets pass through distal tubes 550 and 551 of more flexible material and which have end faces 552 and 553 respectively which are at an acute angle to the longitudinal axis to face laterally outwardly and axially. The angle is about 45°. This configuration may be used for situations where it is particularly desired to direct ventilation gas laterally outwardly for passage into the upper branches off the bronchi. This flow is also assisted by a generally semi-circular cross-sectional shape of the distal tubes 550 and 551.

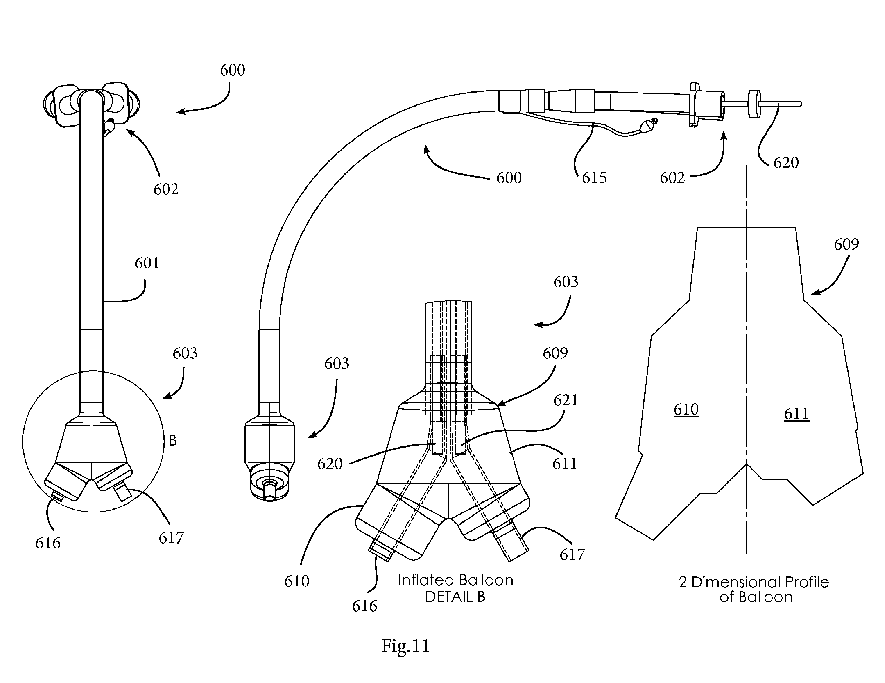

Referring to Fig. 11 a device 600 has a stem 601, a proximal end 602, and a distal end 603. The distal end 603 has a balloon 609 which is non-symmetric in nature, with a longer left branch 610 than right branch 611. This allows entry to the left bronchus more that the right one, to reduce the risk of occluding the right upper lobe of the patient while retaining the carina as a positional anchor. The balloon 609 is manufactured from two thin sheets of polymer material, one of which is placed on top of the other and seam welded around the periphery. Three openings are then left for attachment to the balloon shaft, left and right bronchial legs.

The device 600 also has a pair of stylets 620 and 621 running through distal tubes 616 and 617. In both this embodiment and in the device 500 the stylets provide a desired overall stem curvature, shown in Fig. 11.

The device 600 caters for anatomical variations which would otherwise cause difficulty in placing tubes into the right main bronchus. This is due to the position of the first take off from the right main bronchus, called the right superior lobar bronchus, which can be very close to the start of the right main bronchus, sometimes even starting in the trachea. When the split in the bronchus is close to the start of the right bronchus it is difficult to position a balloon. This device also allows for greater indications for patients with high first take off from the right main bronchus

It will be appreciated that with the invention distal dislocation (with loss of seal between the right/left bronchus and trachea) is very unlikely to occur once the device is placed, particularly as the device does not have a flexible distal element in the bronchus. One balloon held by the tracheal cartilage rings seals the right/left bronchus and trachea, reducing the instance of proximal dislocation

The device maintains constant ventilation cross sectional area from proximal connection to the distal tip - allowing for equal or selective ventilation/aspiration of each lung. There is reduced risk of blockage due to the large distal cross sectional area.

There is ease of insertion, using the carina as a distal location element and reducing the number of movements required. This is in contrast to many prior art double lumen tubes, which require three or four movements, including tracheal placement, rotation to enter bronchus, and bronchial positioning.

During surgery, if proximal dislocation occurs the tube can be re advanced without positional verification (with a bronchoscope) as the carina acts as a location reference (reducing patient risk and time to re position) with clinical assessment of differential ventilation confirming the position.

Also, the outer dimensions of the distal shaft elements may be less than or equal to the trachea shaft, facilitating easy passage through the larynx. The carina offers support to reduce the risk of malposition during insertion and has a high placement success rate on the first attempt. Because the device does not incorporate a dedicated bronchial balloon, rather an integrated trachea/bronchial balloon there is reduced risk of malposition or leakage when moving a patient such as by rolling him or her, collapsing the lung, or manipulating the bronchus.

The device is shorter that standard Double Lumen Tubes (DLT) and will not have the three dimensional curvature required in DLT for placement within the bronchus which will reduced length and curvature, facilitate easy bronchoscope insertion and manipulation. Ventilation of both lungs can be achieved equally during procedures or after procedures. There is fast placement in trauma for initial dual lung ventilation (by inflation of the proximal balloon in the trachea), allowing for later one-lung-ventilation ("OLV") without blocker or tube change-out when using the two balloon embodiment. Also, following elective surgery the device can be withdrawn into the trachea, allowing for dual lung ventilation without tube change out, through inflation of the proximal balloon in the two balloon embodiment.

It will also be appreciated that the device can be repositioned between trauma and OLV and post surgery, there is compatibility with conventional ventilation apparatus connected to the proximal tubes, and there is ease of insertion and positioning with reduced device movement. Furthermore, there is little or no risk of dislocation distally, reduced risk of dislocation proximally, and a consistent cross section from proximal to distal offering selective lung ventilation with one device and easy switching during procedures. It is also advantageous that there is only one device for right and left ventilation, and also there is a low profile on distal end (without need for a retaining sleeve). The integrated balloon arrangement also allows a shorter device length and less curvature than standard Double Lumen Tubes (DLT) facilitating easy bronchoscope insertion and manipulation. Also, the device is particularly suitable for paediatric patients with small anatomy

The invention is not limited to the embodiments described but may be varied in construction and detail.

## Claims

1. A lung ventilation device (1) for human or animal treatment, the device comprising:
a stem (2) configured to fit in a trachea (T),
said stem having at least two ventilation passageways (21, 22) communicating with distal passageways (8, 9) configured to fit in the bronchi (B1, B2) at the carina, and
a positioning balloon (25) configured to engage the walls at the carina when inflated, **characterized in that**:
the positioning balloon (25) has a proximal annular portion (30) surrounding a distal end of the stem, and a plurality of annular distal portions (31, 32), each surrounding a distal passageway end (8, 9), and
the distal balloon portions (31, 32) are extensions of the proximal balloon portion (30).

2. A lung ventilation device as claimed in claim 1, wherein the positioning balloon (25) is configured to, when inflated, seal against the bronchi and the trachea.

3. A lung ventilation device as claimed in claims 1 or 2, wherein each distal balloon portion (31, 32) includes a turned-in wall (27) which is integrated with a wall of the associated distal passageway.

4. A lung ventilation device as claimed in any preceding claim, wherein each ventilation passageway comprises a tube (21, 22), and the balloon distal passageways (8, 9) are continuations of said ventilation passageway tubes (21, 22).

5. A lung ventilation device as claimed in any preceding claim, wherein there is a frangible link (95) between the distal passageways (78, 79), said link being adapted to break to separate the distal passageways *in situ* for bronchi entry.

6. A lung ventilation device as claimed in claim 5, wherein said link (95) is adapted to break as the positioning balloon (85) is inflated.

7. A lung ventilation device as claimed in either of claims 5 or 6, wherein the frangible link (95) is at or adjacent to the apex of an inverted V-shape formed by ends of said distal passageways.

8. A lung ventilation device as claimed in any preceding claim, wherein the device further comprises a stylet (505, 507) extending along the stem (501) and arranged to provide stiffness to the distal passageways (520, 521) during device advancement in the trachea, and wherein there is a stylet (505, 507) for each ventilation passageway (504, 506), the distal passageways (520, 521) have a default splayed-out configuration, and the stylets are arranged to retain the distal passageway together during tracheal advancement and to be retracted for separation at or near the carina.

9. A lung ventilation device as claimed in any preceding claim, wherein the distal passageways (550, 551) have end faces (552, 553) which face outwardly at an acute angle to a longitudinal axis of the stem.

10. A lung ventilation device as claimed in any preceding claim, wherein the balloon (25) is configured so that the action of inflation positions each distal passageway toward the centre of the bronchus.

11. A lung ventilation device as claimed in any preceding claim, wherein one distal balloon portion (610) extends further distally than another distal balloon portion (611).

12. A lung ventilation device as claimed in any preceding claim, wherein the stem includes a lumen (509) for liquid such as radiopaque material to be used during treatment.

13. A ventilation device as claimed in any preceding claim, wherein the proximal end and the stem of the device has a separate passageway corresponding to each distal passageway.

14. A ventilation device as claimed in any preceding claim, wherein the distal passageways (520, 521) are of a material which is more flexible than the stem.

15. A ventilation device as claimed in any preceding claim, wherein the device further comprises a proximal balloon (402) around the stem and being proximal of the positioning balloon (403) and a separate inflation means allowing inflation of the proximal balloon for an initial stage of dual lung inflation before inflation of the positioning balloon for one lung ventilation.

## Patentansprüche

1. Lungenbeatmungsvorrichtung (1) zur Behandlung von Menschen oder Tieren, wobei die Vorrichtung Folgendes umfasst:
einen Schaft (2), der dazu ausgebildet ist, in eine Luftröhre (T) zu passen,
wobei der Schaft mindestens zwei Beatmungsdurchgänge (21, 22) aufweist, die mit distalen Durchgängen (8, 9) in Verbindung stehen, die dazu ausgebildet sind, am Teilungssporn in die Bronchien (B1, B2) zu passen, und
einen Positionierungsballon (25), der dazu ausgebildet ist, wenn er aufgeblasen ist, mit den Wänden am Teilungssporn in Eingriff zu stehen,
**dadurch gekennzeichnet, dass**:
der Positionierungsballon (25) einen proximalen kreisringformigen Abschnitt (30), der ein distales Ende des Schafts umgibt, und mehrere kreisringförmige distale Abschnitte (31, 32), die jeweils ein distales Durchgangsende (8, 9) umgeben, aufweist, und
es sich bei den distalen Ballonabschnitten (31, 32) um Erweiterungen des proximalen Ballonabschnitts (30) handelt.

2. Lungenbeatmungsvorrichtung nach Anspruch 1, wobei der Positionierungsballon (25) dazu ausgebildet ist, wenn er aufgeblasen ist, gegen die Bronchien und die Luftröhre zu dichten.

3. Lungenbeatmungsvorrichtung nach Anspruch 1 oder 2, wobei die distalen Ballonabschnitte (31, 32) jeweils eine eingedrehte Wand (27) umfassen, die mit einer Wand des zugehörigen distalen Durchgangs integriert ist.

4. Lungenbeatmungsvorrichtung nach einem der vorangehenden Ansprüche, wobei die Beatmungsdurchgänge jeweils eine Röhre (21, 22) umfassen und es sich bei den distalen Durchgängen (8, 9) des Ballons um Fortsetzungen der Beatmungsdurchgangsröhren (21, 22) handelt.

5. Lungenbeatmungsvorrichtung nach einem der vorangehenden Ansprüche, wobei eine brüchige Verbindung (95) zwischen den distalen Durchgängen (78, 79) vorliegt, wobei die Verbindung dazu angepasst ist, zu brechen, um die distalen Durchgänge zum Eindringen in die Bronchien in situ zu trennen,

6. Lungenbeatmungsvorrichtung nach Anspruch 5, wobei die Verbindung (95) dazu angepasst ist, zu brechen, während der Positionierungsballon (85) aufgeblasen wird.

7. Lungenbeatmungsvorrichtung nach einem der Ansprüche 5 oder 6, wobei sich die brüchige Verbindung (95) an oder benachbart der Spitze einer umgekehrten V-Form befindet, die von Enden der distalen Durchgänge gebildet wird.

8. Lungenbeatmungsvorrichtung nach einem der vorangehenden Ansprüche, wobei die Vorrichtung weiter einen Führungsstab (505, 507) umfasst, der sich entlang dem Schaft (501) erstreckt und dazu angeordnet ist, den distalen Durchgängen (520, 521) während des Voranbewegens der Vorrichtung in der Luftröhre Steifigkeit zu verleihen und wobei für jeden Beatmungsdurchgang (504, 506) ein Führungsstab (505, 507) vorliegt, wobei die distalen Durchgänge (520, 521) eine vorgegebene gespreizte Ausbildung aufweisen und die Führungsstäbe dazu angeordnet sind, den distalen Durchgang während des Voranbewegens in der Luftröhre zusammenzuhalten und zur Trennung an oder nah beim Teilungssporn zurückgezogen zu werden.

9. Lungenbeatmungsvorrichtung nach einem der vorangehenden Ansprüche, wobei die distalen Durchgänge (550, 551) Stirnflächen (552, 553) aufweisen, die unter einem spitzen Winkel zu einer Längsachse des Schafts nach außen weisen.

10. Lungenbeatmungsvorrichtung nach einem der vorangehenden Ansprüche, wobei der Ballon (25) derart ausgebildet ist, dass die Handlung des Aufblasens jeden distalen Durchgang zur Mitte der Bronchie hin positioniert.

11. Lungenbeatmungsvorrichtung nach einem der vorangehenden Ansprüche, wobei sich ein distaler Ballonabschnitt (610) weiter distal erstreckt als ein anderer distaler Ballonabschnitt (611).

12. Lungenbeatmungsvorrichtung nach einem der vorangehenden Ansprüche, wobei der Schaft ein Lumen (509) für während der Behandlung zu verwendende Flüssigkeit, wie etwa röntgendichtes Material, umfasst.

13. Beatmungsvorrichtung nach einem der vorangehenden Ansprüche, wobei das proximale Ende und der Schaft der Vorrichtung einen jedem distalen Durchgang entsprechenden getrennten Durchgang aufweist.

14. Beatmungsvorrichtung nach einem der vorangehenden Ansprüche, wobei die distalen Durchgänge (520, 521) aus einem Material sind, das biegsamer ist als der Schaft.

15. Beatmungsvorrichtung nach einem der vorangehenden Ansprüche, wobei die Vorrichtung weiter einen proximalen Ballon (402) um den Schaft umfasst, der proximal zum Positionierungsballon (403) ist, und ein getrenntes Aufblasmittel umfasst, das das Aufblasen des proximalen Ballons für eine anfängliche Stufe des Aufblasens von zwei Lungen vor dem Aufblasen des Positionierungsballons zum Beatmen einer Lunge ermöglicht.

## Revendications

1. Dispositif de ventilation pulmonaire (1) à des fins de traitement chez l'homme ou chez l'animal, le dispositif comportant :
une tige (2) configurée à des fins d'ajustement dans une trachée (T),
ladite tige ayant au moins deux voies de passage de ventilation (21, 22) en communication avec des voies de passage distales (8, 9) configurées à des fins d'ajustement dans les bronches (B1, B2) au niveau de la carina, et
un ballonnet de positionnement (25) configuré à des fins de mise en prise avec les parois au niveau de la carina quand il est gonflé, **caractérisé en ce que** :
le ballonnet de positionnement (25) a une partie annulaire proximale (30) entourant une extrémité distale de la tige, et une pluralité de parties distales annulaires (31, 32), chacune entourant une extrémité de voie de passage distale (8, 9), et
les parties de ballonnet distales (31, 32) sont des extensions de la partie de ballonnet proximale (30).

2. Dispositif de ventilation pulmonaire selon la revendication 1, dans lequel le ballonnet de positionnement (25) est configuré pour, quand il est gonflé, venir se sceller contre les bronches et la trachée.

3. Dispositif de ventilation pulmonaire selon la revendication 1 ou la revendication 2, dans lequel chaque partie de ballonnet distale (31, 32) comprend une paroi retournée vers l'intérieur (27) qui fait partie intégrante d'une paroi de la voie de passage distale associée.

4. Dispositif de ventilation pulmonaire selon l'une quelconque des revendications précédentes, dans lequel chaque voie de passage de ventilation comporte un tube (21, 22), et les voies de passage distales (8, 9) du ballonnet sont des continuations desdits tubes des voies de passages de ventilation (21, 22).

5. Dispositif de ventilation pulmonaire selon l'une quelconque des revendications précédentes, dans lequel une liaison cassable (95) se trouve entre les voies de passage distales (78, 79), ladite liaison étant adaptée pour casser afin de séparer les voies de passage distales *in situ* à des fins d'entrée dans les bronches.

6. Dispositif de ventilation pulmonaire selon la revendication 5, dans lequel ladite liaison (95) est adaptée pour casser quand le ballonnet de positionnement (85) est gonflé.

7. Dispositif de ventilation pulmonaire selon l'une quelconque des revendications 5 ou 6, dans lequel la liaison cassable (95) se trouve au niveau du sommet, ou de manière adjacente par rapport au sommet, d'une forme en V renversé formée par des extrémités desdites voies de passage distales.

8. Dispositif de ventilation pulmonaire selon l'une quelconque des revendications précédentes, dans lequel le dispositif comporte par ailleurs un stylet (505, 507) s'étendant le long de la tige (501) et agencé à des fins de mise en oeuvre d'une rigidité au niveau des voies de passage distales (520, 521) au cours de l'avancement du dispositif dans la trachée, et dans lequel il y a un stylet (505, 507) pour chaque voie de passage de ventilation (504, 506), les voies de passage distales (520, 521) ont une configuration évasée vers l'extérieur par défaut, et les stylets sont agencés pour retenir la voie de passage distale ensemble au cours de l'avancement dans la trachée et pour être rétractés à des fins de séparation au niveau ou à proximité de la carina.

9. Dispositif de ventilation pulmonaire selon l'une quelconque des revendications précédentes, dans lequel les voies de passage distales (550, 551) ont des faces d'extrémité (552, 553) qui sont orientées vers l'extérieur selon un angle aigu par rapport à un axe longitudinal de la tige.

10. Dispositif de ventilation pulmonaire selon l'une quelconque des revendications précédentes, dans lequel le ballonnet (25) est configuré de telle sorte que l'action de gonflage positionne chaque voie de passage distale vers le centre de la bronche.

11. Dispositif de ventilation pulmonaire selon l'une quelconque des revendications précédentes, dans lequel une partie de ballonnet distale (610) s'étend plus encore dans le sens distal par rapport à une autre partie de ballonnet distale (611).

12. Dispositif de ventilation pulmonaire selon l'une quelconque des revendications précédentes, dans lequel la tige comprend une lumière (509) pour liquide tel qu'un produit de contraste à des fins d'utilisation en cours de traitement.

13. Dispositif de ventilation selon l'une quelconque des revendications précédentes, dans lequel l'extrémité proximale et la tige du dispositif ont une voie de passage séparée qui correspond à chaque voie de passage distale.

14. Dispositif de ventilation selon l'une quelconque des revendications précédentes, dans lequel les voies de passage distales (520, 521) sont réalisées à partir d'une matière qui est plus flexible que la tige.

15. Dispositif de ventilation selon l'une quelconque des revendications précédentes, dans lequel le dispositif comporte par ailleurs un ballonnet proximal (402) autour de la tige, et étant proximal par rapport au ballonnet de positionnement (403) et un moyen de gonflage séparé permettant le gonflage du ballonnet proximal pour une étape initiale de gonflage pulmonaire double avant le gonflage du ballonnet de positionnement pour une ventilation d'un poumon.
